# EUROPÄISCHE PATENTSCHRIFT

(11) **EP 4 260 714 B1**
(45) Veröffentlichungstag und Bekanntmachung des Hinweises auf die Patenterteilung: **08.07.2026**
(21) Anmeldenummer: 23166935.9
(22) Anmeldetag: 06.04.2023
(51) Int. Cl.: A23L 25/10, A23L 25/00, A23G 3/34

(54) **VERFAHREN ZUM HERSTELLEN EINER PUMPBAREN PASTE AUS DEN SAMEN VON NUSS- ODER STEINFRÜCHTEN**
METHOD FOR PRODUCING A PUMPABLE PASTE FROM THE SEEDS OF NUT OR STONE FRUITS
PROCÉDÉ DE FABRICATION D'UNE PÂTE POMPABLE À PARTIR DES GRAINES DE NOIX OU FRUITS À NOYAU

(30) Priorität: 14.04.2022 DE 102022109275
(43) Veröffentlichungstag der Anmeldung: 18.10.2023
(73) Patentinhaber: NETZSCH-Feinmahltechnik GmbH, 95100 Selb (DE)
(72) Erfinder: Weidgenant, Matheus, Selb (DE); Kapp, Matthias, Weilersbach (DE)
(74) Vertreter: Misselhorn, Hein-Martin

(56) Entgegenhaltungen:
- EP-A1- 2 446 748
- US-A- 3 592 662
- US-A- 5 591 477
- US-A1- 2003 211 224
- US-A1- 2010 127 108
- US-A1- 2018 318 840
- US-A1- 2019 373 910
- US-A1- 2020 297 013
- US-B2- 11 235 335
- ANONYMOUS: "Fragen und Antworten | Betty Butter", HTTPS://WWW.BETTYBUTTER.DE/FRAGEN-UND-ANTWORTEN, 7 August 2020 (2020-08-07), pages 1 - 2, XP093068549, Retrieved from the Internet <URL:https%3A%2F%2Fwww.bettybutter.de%2Ffragen-und-antworten> [retrieved on 20230728]

## Beschreibung

Die Erfindung betrifft ein Verfahren nach dem Oberbegriff des Anspruchs 1

### TECHNISCHER HINTERGRUND

Von je her erfreuen sich mandelhaltige Erzeugnisse wie darunter insbesondere mandelhaltige Confiseriewaren, wie etwa Torten, Kuchen, Pralineés, Petit Fours, Speiseeis, Marzipan und mandelhaltiges Kleingebäck hoher Beliebtheit. Ganze oder gehobelte Mandeln kommen dabei meist nur zur Dekorationszwecken zum Einsatz. Weitaus häufiger wird für solche Produkte Mandelmasse benötigt.

Darüber hinaus finden mandelhaltige Erzeugnisse seit langem im Kosmetikbereich Verwendung, nicht nur in Gestalt von hautpflegendem Mandelöl oder hautreinigender Mandelkleie sondern immer wieder auch Gestalt fein gemahlener, hautpflegender Mandelpasten.

Der Bedarf an mandelhaltigen Erzeugnissen hat in den vergangenen Jahren mit dem starken Aufschwung der veganen Bewegung und dem gleichzeitig verstärkten Diagnostiziertwerden von Laktoseintoleranzen und Milcheiweißallergien sprunghaft zugenommen. Das ist nicht zuletzt darauf zurückzuführen, dass sich mit Hilfe sehr fein gemahlener Mandelpasten und des darin aufgeschlossenen Eiweißes und Fettes unter Zugabe von Wasser eine Emulsion herstellen lässt die optisch, sensorisch und geschmacklich an Milch erinnert und daher zunehmend als entsprechendes Substitut Absatz findet.

Da Mandelmilch "parve" (neutral, also weder milch- noch fleischhaltig) ist, besitzt sie zudem auch im Kontext mit dem Kaschrut, d. h. dem jüdischen Speisegesetz einige Relevanz.

Was im allgemeinen Sprachgebrauch als "Mandel" bezeichnet wird, ist der Samen der eine sog. Steinfrucht ausbildenden Mandelfrucht. Bei ihr wird der Samen von einem verholzten Kern, dem sog. Mandelkern umschlossen.

In den bis hierher geschilderten Einsatzfeldern kommen aber nicht nur Steinfrüchte, wie die Mandelfrucht bzw. deren Mandel zum Einsatz, sondern in ähnlichem Umfang auch die Nussfrüchte, also Früchte bei denen der Samen von einer hart verholzten Außenschale umschlossen wird.

Bei alledem werden Produkte aus den Samen der Stein- oder Nussfrüchte nicht zuletzt von Seiten der industriellen Lebensmittelerzeugung als fein gemahlene, pumpbare Zwischenprodukte nachgefragt, die als Ansatzmassen bezeichnet werden.

Ein pumpbares Zwischenprodukt der genannten Art lässt sich in all jenen Fällen leicht herstellen, in denen es nicht stört, wenn das fertige Zwischenprodukt ihm zur Verbesserung seiner Fließfähigkeit zugegebenes Wasser oder Öl anderer Provenienz und eventuell auch noch nicht-steinfruchteigene oder nichtnussfruchteigene Emulgatoren enthält.

Aus geschmacklichen Gründen und/oder aus Gründen besserer Verbraucherakzeptanz soll das jeweilige Zwischenprodukt jedoch zunehmend ohne Zusatzstoffe auskommen, die nicht von den Schließfrüchten bzw. deren Samen herrühren. Auch die Zugabe von Wasser als Fließfähigkeitsverbesserer ist nicht unproblematisch, da jeder Wasserzugabe mit der Gefahr eines Eintrags von Keimen einhergeht. Selbst wenn kein Keimeintrag über das Wasser erfolgt begünstigt ein höherer Wasseranteil das Wachstum von eventuell von im Zwischenprodukt aus anderen Gründen befindlichen Keimen.

Daher haben sich in der Vergangenheit für die Verarbeitung von Mandeln oder Nüssen etabliert, die sich einer Röstung der Mandeln oder Nüsse bedienen, an die sich die Vermahlung der Mandeln oder Nüsse anschließt. Durch die Röstung wird der natürliche Wassergehalt der Mandeln oder Nüsse reduziert. Dabei lässt die Rösthitze die Zellwände aufbrechen und setzt dadurch das im Inneren der jeweiligen Zelle gespeicherte Fett frei. Dadurch erhält man unmittelbar im Anschluss an das Rösten eine pumpbare Masse die bequem weiterverarbeitet werden kann.

Mit dem Rösten geht allerdings fast immer eine mehr oder minder ausgeprägte Farbveränderung ins Bräunliche einher. Eine solche Farbveränderung stört nicht zuletzt bei der Produktion von Milchersatzprodukten, die in vielen Fällen möglichst das von Milchprodukten her vertraute weiß zeigen sollen.

Außerdem geht mit dem Rösten der Mandeln oder Nüsse nahezu unweigerlich auch eine mehr oder minder ausgeprägte MaillardReaktion einher, also eine komplexe Gesamtheit nebeneinander und (oder nacheinander ablaufende Reaktionen, die zu einer Vielzahl von Reaktionsprodukten führt, die den Geschmack beeinflussen bzw. von den Verbrauchern abgelehnt werden, da bekannt ist, dass unter diesen Reaktionsprodukten - jedenfalls bei nur suboptimaler Verfahrensführung - mutagene oder karzinogene Reaktionsprodukte zu finden sein können.

Die US 2020/297013 A1 betrifft eine Nusspastenzubereitung für Lebensmittel und Getränke, die durch Verarbeitung blanchierter, ungerösteter Nüsse zu einem Mehl auf Nussbasis mit einer mittleren Teilchengröße zwischen etwa 0,002 und 0,012 Zoll hergestellt wird. Die Verarbeitung erfolgt ohne Zugabe von Wasser und Öl. Die Temperatur während der Verarbeitung liegt nicht über 140 Grad Fahrenheit. Das Mehl auf Nussbasis wird ohne Zugabe von Wasser und Öl geschert, um eine Nusspaste zu bilden. Die Temperatur während des Scherens überschreitet nicht 120 Grad Fahrenheit. Die Nusspaste hat nach dem Scheren eine mittlere Teilchengröße von etwa 1 bis etwa 40 Mikrometer.

Die US 2003/211224 A1 betrifft ein essbares Produkt, das eine quetschbare Nussbutter in Kombination mit einer weiteren essbaren Komponente umfasst. Bei der Komponente handelt es sich im Allgemeinen um eine andere Lebensmittelkomponente, die vorzugsweise mit Erdnussbutter gegessen wird, wie etwa Gelee, Banane, Marshmallowfüllung, Schokolade, Speckstückchen usw.

Die US 3 592 662 A betrifft ein Verfahren zur Behandlung eines zerkleinerten Erdnussbreis. Die EP 2 446 748 A1 betrifft ein Verfahren zur Herstellung einer Schokoladenmasse und Vorrichtung dafür. Die US 2018/318840 A1 betrifft eine Zerkleinerungsvorrichtung und Verfahren zum Zerkleinern von Rohstoffen. Die Publikation Anonymous: "Fragen und Antworten | Betty Butter", https://bettybutter.de/fragen-und-antworten, 7. August 2020 (2020-08-07), Seiten 1-2, XP093068549, Gefunden im Internet: URL: https%3A%2F%2Fwww.bettybutter.de%2Ffragen-und-antworten [gefunden am 2023-07-28] betrifft die Herstellung von Nussmus. Die US 2019/373910 A1 betrifft eine Conchiervorrichtung und ein Verfahren zum Conchieren einer Produktmasse. Die US 2010/127108 A1 betrifft eine Rührwerkskugelmühle. Die US 5 591 477 A1 betrifft ein Verfahren zur Herstellung einer fettreduzierten Erdnussbutter ohne Nicht-Erdnusszusätze und ein daraus hergestelltes Produkt.

### AUFGABE DER ERFINDUNG

Demgegenüber ist es die Aufgabe der Erfindung ein Verfahren zur Herstellung einer pumpfähigen Ansatzmasse aus gemahlenen Steinfrucht- oder Nussfruchtsamen anzugeben, das ohne die Zugabe von Nicht-Stein- oder Nicht-Nussfruchtsamen-Bestandteilen auskommt und keinen oder nur einen geringen Einfluss auf den Geschmack der Ansatzmasse hat.

### DIE ERFINDUNGSGEMÄSSE LÖSUNG

Die Erfindung wird durch den Gegenstand des unabhängigen Anspruchs 1 definiert. Vorteilhafte Weiterbildungen sind Gegenstand der abhängigen Ansprüche.

### WEITERE MERKMALE UND VORTEILE DER ERFINDUNG

Die erfindungsgemäße Lösung ist ein Verfahren zur Zubereitung einer pumpfähigen Ansatzmasse aus Steinfrucht- oder/und Nussfruchtsamen, insbesondere Nüssen, Mandeln oder Nuss- und Mandelgemischen, mit folgenden Schritten: Zunächst erfolgt ein Aufbrechen der bereits ausgesteinten oder ausgeschalten Steinfrucht- oder Nussfruchtsamen durch Vorzerkleinerung.

Anschließend erfolgt eine Heißluft- und/oder Vakuumexposition der durch die Vorzerkleinerung entstandenen Masse zum Zwecke des Aufbrechens ihrer Zellen in einer Conchiervorrichtung (8) mit einem zumindest im Wesentlichen abgeschlossenen Conchierbehälter (9) und einem darin bevorzugt an einer horizontalen Welle umlaufenden, meist mehrarmigen Conchierwerkzeug.

Hieran schließt sich eine meist einstufige, ggf. auch mehrstufige Weitervermahlung der durch die Vorvermahlung und die Heißluft- bzw. Vakuumexposition entstandenen Masse zu einer pumpfähigen Ansatzmasse an.

Durch die Vorzerkleinerung wird die freie Oberfläche der im Anschluss dem Vakuum und/oder der Heißluft ausgesetzten Steinfrucht- oder Nussfruchtsamen (sog. Vorzerkleinerungsgut) um ein Vielfaches erhöht. Auf diese Art und Weise wird es möglich durch Anlegen eines Vakuums und/oder durch Anlegen von Heißluft, die Zellwände der überwiegend noch intakt gebliebenen Zellen des Vormahlgutes zum Aufplatzen zu bringen. Hierdurch werden die in den intakten Zellen gespeicherten Fette oder Ölbestandteile aufgeschlossen. Die Fette bzw. Ölbestandteile werden derart mobilisiert, dass sie die Viskosität des Vorzerkleinerungsgutes wesentlich erhöhen und damit das Vorzerkleinerungsgut pumpbar machen.

Unter einer "Ansatzmasse" wird dabei eine Masse aus den hier in Rede stehenden Naturprodukten verstanden, die als kommerzielle Produkt und entsprechend Konfektioniert zum Bedarfsträger gebracht, ggf. gelagert und bei Bedarf - bevorzugt mit Hilfe von Dosierpumpen - weiteren Ingredienzen zugemischt und zusammen damit zu Fertigprodukten weiterverarbeitet werden kann, wie etwa Gebäck, Speiseeis oder Pflegeprodukten. Primärzweck der Anatzmasse kann es sein den Fertigprodukten des charakteristischen Geschmack der Rohprodukte zu verleihen aus denen die Ansatzmasse besteht.

### BEVORZUGTE WEITERE AUSGESTALTUNGSMÖGLICHKEITEN

Bevorzugterweise wird die Heißluft- bzw. Vakuumexposition insbesondere über die Expositionsdauer so gesteuert, dass die Restfeuchte des Vorzerkleinerungsgutes auf unter 2,1% und bevorzugt auf unter 1,85% reduziert wird. Auch zur Erreichung dieses Ziels trägt die das Verfahren einleitende bzw. vorangehende Vorzerkleinerung wesentlich bei. Denn durch das dadurch begünstigte Aufplatzen der Zellwände unter dem Einfluss des Vakuums oder der Heißluft wir neben den Fett- und Ölbestandteilen auch der in den Zellen gespeicherte Wasseranteil freigesetzt und kann nun leicht abgezogen werden. Die Verringerung des Wasseranteils trägt wesentlich zur besseren Haltbarkeit der fertigen Ansatzmasse bei, da Massen mit einen möglichst geringen Wasseranteil ein schlechter Nährboden für Sporen und Keime sind. Da erfindungsgemäß die gewünschte Pumpfähigkeit über die Mobilisierung der bislang in den Zellen eingeschlossenen Fett- und Ölbestandteile erfolgt behindert der weitere Wasserentzug das Erreichen der gewünschten Pumpfähigkeit nicht.

Vorzugsweise liegt die Lufttemperatur der zum Trocknen eingesetzten Heißluft im Bereich von oberhalb von 60° C und idealerweise im Bereich von oberhalb 75° C. Erst bei diesen Temperaturen kommt es zu einem hinreichend schnellen, praktisch sinnvolle Prozessdauern gewährleistenden Aufplatzen der Zellwände.

Besonders bevorzugt ist, dass die Lufttemperatur der zum Trocknen eingesetzten Heißluft im Bereich von unterhalb von 100° C und idealerweise im Bereich von unterhalb 90° C liegt. Damit lieg die Prozesstemperatur unter bzw. deutlich unter der Schwelle von etwa 140° C, ab der die Maillard-Reaktionen abzulaufen beginnen, also "Röstprozesse" stattfinden. Somit entstehen im Zuge der Heißluftapplikation weder Verfärbungen noch die für das Rösten typischen, in anderen Zusammenhängen als hier durchaus gewünschten Röstaromen

Vorzugsweise wird darauf geachtet, dass die zum Trocknen eingesetzten Heißluft den Trocknungsbereich derart durchströmt, dass sie von dort noch während des weiter voranschreitenden Trocknungsprozesses abgeführt wird, sobald sie gemessenermaßen oder erfahrungsgemäß eine bestimmte Menge Feuchtigkeit aufgenommen hat.

Soweit statt der Heißluft oder flankierend zu ihr ein Vakuum angelegt wird um das Aufplatzen der Zellen zu erreichen oder zu intensivieren und/oder die Verringerung der Restfeuchte zu erreichen, kommt bevorzugt ein Vakuum mit einen Absolutdruck von weniger als 0,25 bar und idealerweise im Bereich von 0,1 bar zur Anlage.

Im Regelfall, von eventuellen Ausnahmen abgesehen, wird bei der Vorzerkleinerung und im Regelfall auch bei der Weitervermahlung kein Wasser, Öl oder sonstiger Steinfrucht- oder Nussfruchtsamenfremder Stoff zugesetzt. Das spiegelt gerade die Stärke des erfindungsgemäßen Verfahrens wieder. Durch die Heißluft- oder Vakuumexposition und den damit einhergehenden Aufschluss der in den Zellen gespeicherten Fette und Öle wird schon das Vorzerkleinerungsgut pumpfähig. Dadurch wird es möglich das Vorzerkleinerungsgut ohne weitere Zusatzstoffe und insbesondere ohne Wasser- und/oder Ölzugabe durch eine Rührwerkskugelmühle hindurchzupumpen. Dadurch wird eine sehr effektive und genau abgestimmte Weiterzerkleinerung der Bestandzeile, die die Ansatzmasse bilden möglich, oft einhergehend mit einer weiteren Erhöhung der Pumpfähigkeit der Ansatzmasse.

Es ist in besonderem Maß bevorzugt, wenn die Heißluft- oder Vakuumapplikation in einem Horizontaltrommelmischer stattfindet, der die vorvermahlenden Steinfrucht- oder Nussfruchtsamen in permanenter Bewegung hält. Auf diese Art kommt die Masse aus den vorvermahlenen Steinfrucht- oder Nussfruchtsamen auch dann besonders intensiv mit der Heißluft oder dem Vakuum in Kontakt wenn sie schon dazu neigt unter dem Einfluss ersten Fett-, Öl, oder Wasseraustritts aus den Zellen zu klumpen, was insbesondere dann der Fall ist, wenn die Masse bereits durch die Vorzerkleinerung in deutlich kleinere Partikel zerlegt worden ist, als z. B. bloße Nuss- oder Mandelsplitter.

Die Heißluft- oder Vakuumapplikation findet in einer Conchiervorrichtung mit einem zumindest im Wesentlichen abgeschlossenen Conchierbehälter und einem darin bevorzugt an einer horizontalen Welle umlaufenden, meist mehrarmigen Conchierwerkzeug durchzuführen. Conchiervorrichtungen sind aus der Schokoladenherstellung bekannt. Sie sind dazu in der Lage auch solche Massen zu belüften und intensiv mit Heißluft oder Vakuum interagieren zu lassen, die auf Grund ihres recht hohen freien Fettanteils zunächst noch klebrig/zäh sind und daher erst einmal noch nicht pumpfähig sind.

Bevorzugt erfolgt die Vorzerkleinerung durch eine Vorzerkleinerungsvorrichtung in Gestalt eines Mahlwerks insbesondere eines Kegelmahlwerks. Ein solches weist einen mit einer - bevorzugt vertikal angeordneten - Welle umlaufenden Brechkegel auf, der in unter Ausbildung eines Brechspalts einem stationären Brechring umläuft. Dabei tragen die den Brechspalt zwischen sich ausbildenden Flächen typischerweise eine Mitnehmer bildende Profilierung. Diese dient zum Einzug der vorzuzerkleinernden Steinfrucht- oder Nussfruchtsamen in den Brechspalt und sorgt dafür dass die Steinfrucht- oder Nussfruchtsamen sich formschlüssig im Brechspalt "verklemmen" und dann im Zuge des Weiterdrehens des Brechkegels zertrümmert werden. Dadurch dass mit einem Brechspalt gearbeitet wird und sich der Brechkegel und der Brechring nirgendwo berühren wird in jedem Fall nicht so weit vorgebrochen, das sich der Brechspalt zusetzen kann. Zudem lässt sich die kann die Feinheit der Partikel, die das Ergebnis des Vorbrechens sind gut einstellen, indem der Abstand verändert wird, den der Mahlkegel voneinander einhalten.

Die Heißluft- oder Vakuumapplikation erfolgt in einer Conchiervorrichtung mit einem zumindest im Wesentlichen abgeschlossenen Conchierbehälter und einem darin bevorzugt an einer horizontalen Welle umlaufenden, meist mehrarmigen Conchierwerkzeug.

Nachdem die durch die Vorzerkleinerung und Heißluft- bzw. Vakuumapplikation gewonnene Masse pumpfähig geworden ist erfolgt die Feinvermahlung mit Hilfe einer Rührwerkskugelmühle erfolgt - durch die die Masse nun hindurchgepumpt werden kann. Die Feinvermahlung mit einer Rührwerksmühle führt zu einer sehr gleichmäßigen und feinen Vermahlung, die sich gut steuern und effektiv durchführen lässt Unabhängig von den Verfahrensansprüchen als solche wird auch Schutz für eine Vorrichtung zur Durchführung des Verfahrens nach einem der vorhergehenden Ansprüche beansprucht. Die Vorrichtung zeichnet sich dadurch aus, dass sie einen Vorzerkleinerer (2), eine Conchiervorrichtung (8), einen Heißluft- oder Vakuumapplikaton (13) und eine Feinvermahlungseinrichtung (11) umfasst.

Der Vorzerkleinerer ist so ausgestattet und justiert, dass er die zu behandelnden Steinfrucht oder Nussfruchtsamen derart vorzerkleinert, das aus ihnen mit Hilfe des Heißluft- und/oder Vakuumapplikators eine pumpfähige Masse hergestellt werden kann ohne die Masse bis in den Bereich der Maillardreaktion hinein zu erhitzen. Diese Masse kann dann ohne weitere Zusätze zur Erhöhung der Fließfähigkeit durch eine Rührwerkskugelmühle gepumpt werden.

### FIGURENLISTE

Die Figur 1 zeigt eine erfindungsgemäße Anlage für kleinere Mengen, bei der nur ein Horizontalmischer zum Einsatz kommt in dem Vakuum appliziert wird.
Die Figur 2 zeigt einen Querschnitt durch ein für dieses Ausführungsbeispiel zum Zwecke der Vorzerkleinerung einsetzbares Mahlwerk.

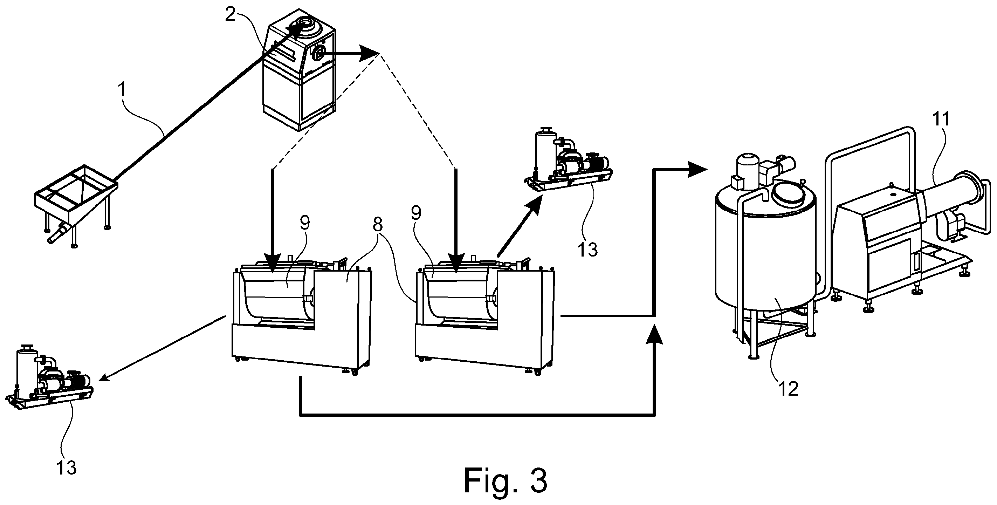

Die Figur 3 zeigt eine erfindungsgemäße Anlage für größere Mengen. Sie zeichnet sich durch zwei parallel arbeitender Horizontalmischer aus, in beiden wird Vakuum appliziert.

### AUSFÜHRUNGSBEISPIELE

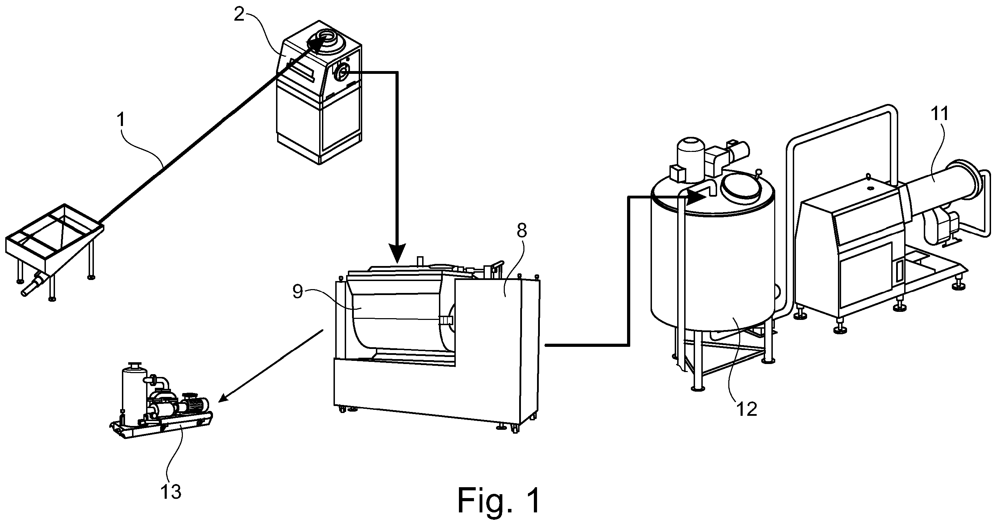

Die Figur 1 zeigt ein erstes Ausführungsbeispiel der Erfindung, hier am Beispiel von Hasel und/oder Cashewnüssen und Mandeln. Das geschilderte gilt aber entsprechend auch für andere Nussamen oder Steinfruchtsamen.

Die besagten Nüsse oder Mandeln werden ganz oder gehobelt über einen Förderer 1, meist ein Schraubenförderer oder ein Vibrationsförderer, auf den Vorzerkleinerer 2 aufgegeben. Als Vorzerkleinerer 2 kann beispielsweise ein Gerät eingesetzt werden wie er von der Firma Netzsch unter der Bezeichnung Mastercream^{®} vertrieben wird.

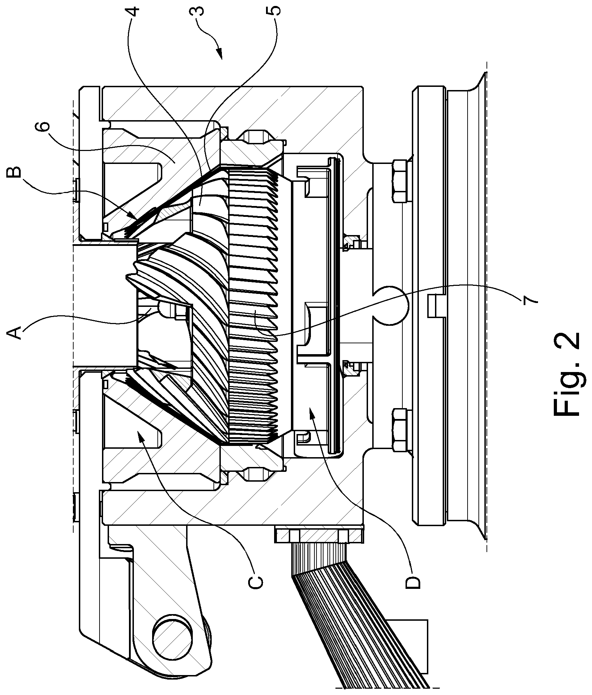

Dieser Vorzerkleinerer ist idealerweise als Kegelmahlwerk 3 ausgebildet, etwa so, wie das die Figur 2 zeigt. Das Kegelmahlwerk 3 wird in vertikaler Richtung von oben her beschickt. Die vorzuzerkleinernden Nüsse bzw. Mandeln fallen auf den sich nach oben hin verjüngenden Brechkegel 4. Der Brechkegel 4 besitzt eine vorzugsweise helikale Profilierung. Diese helikale Profiliierung ergreift die ankommenden Nüsse und zieht sie in den Brechspalt 5. Diesen Brechspalt bildet der Brechkegel 4 zusammen mit dem stationären Brechring 6, in dem er rotiert. Die Rotation erfolgt zusammen mit einer vertikalen Welle. Auf diese Art und Weise werden die vorzuzerkleinernden Nüsse oder Mandeln im Brechspalt zertrümmert. Von dort aus fallen sie in den zweiten Teil des Mahlwerks. Dieser zweite Teil wird durch eine sich an den unteren Rand des Mahlkegels anschließende, zylindrische Mahlscheibe 7 gebildet. Auch die zylindrische Mahlscheibe 7 trägt eine Profilierung die das Mahlen unterstützt.

Der Mahlprozess wird typischerweise so geführt, dass die Temperatur der vorzuzerkleinernden Nüsse bzw. Mandeln unter 110° bleibt, über den gesamten Vorzerkleinerungsvorgang hinweg. Das verhindert eine geschmackliche Beeinflussung oder gar die Bildung von Röststoffen. In den meisten Fällen kann auf eine Wasserkühlung des Vorzerkleinerers und seines Mahlwerks verzichtet werden, stattdessen wird über eine geeignete Einstellung der Spaltweite des Mahlwerks und oder von dessen Wellendrehzahl erreicht, das keine übermäßige Erwärmung stattfindet.

Der Vorzerkleinerer 2 wird bevorzugt mit einer Wellengeschwindigkeit von etwa 3000 bis 4200 U/min betrieben. Entsprechend schnell rotiert der Brechkegel 4.

Im Vorzerkleinerer entsteht eine zähe Masse aus Nuss- bzw. Mandelpaste. Die hierin enthaltenen Partikel zeigen überwiegend einen durchschnittlichen Partikeldurchmesser im Bereich von 30/100 mm bis etwa 4/100 mm.

Obwohl in dieser Masse schon ein Teil der in den Zellen gespeicherten Öle und Fette mobilisiert worden ist, ist die Masse nicht pumpfähig. Wählt man den lichten Durchmesser der Verbindungsleitung nicht zu klein und führt die Verbindungsleitung kurz aus, dann kann die fertig vorzerkleinerte Masse unter dem Einfluss der Schwerkraft ohne weitere Hilfsmittel nach unten in den tiefer angeordneten Horizontalmischer hineinfallen.

Als Horizontalmischer wird eine Conchiervorrichtung 8 eingesetzt, wie man sie von der Schokoladenherstellung her kennt.

Eine solche Conchiervorrichtung 8 kann ähnlich gestaltet sein wie beispielsweise von der Anmelderin in ihrem deutsche Patent DE 10 2017 001 784 B4 beschriebene Conchiervorrichtung.

Eine Conchiervorrichtung zeichnet sich durch einen Conchierbehälter 9 aus. In diesem läuft eine horizontale Welle um. An der Welle ist im Regelfall ein mehrarmiges Conchierwerkzeug 10 befestigt. Dessen Arme pflügen die zähe Masse, die durch die Vorzerkleinerung entstanden ist, buchstäblich bei jeder Umdrehung um. Auf diese Art und Weise wird die Masse sehr gleichmäßig dem im Conchierbehälter anzutreffenden Vakuum oder der dort anzutreffenden heißen Luft ausgesetzt, das oder die in der Conchiervorrichtung appliziert wird.

Der Conchierbehälter ist typischerweise an eine Vakuumpumpe angeschlossen. Die Vakuumpumpe evakuiert den Conchierbehälter teilweise. Sie setzt diesen also unter Unterdruck.

Das erfolgt vorzugsweise derart, dass in dem Conchierbehälter weniger als 25 % des natürlichen Umgebungsdrucks herrscht, idealerweise herrscht ein Unterdruck im Bereich von 0,1 bar, absolut.

Die Conchiervorrichtung arbeitet im Chargenbetrieb. Sie wird vom Vorzerkleinerer solange beschickt, bis sie ihre Nennbeladung erreicht hat. Damit der Vorzerkleinerungsprozess gestoppt. Nun beginnt die Conchiervorrichtung zu arbeiten.

Gerade auch bei der Anwendung von Vakuum hat es sich als zweckmäßig erwiesen, wenn die Conchiervorrichtung mit in separaten Kanälen geführtem und daher nicht mit dem zu conchierenden Produkt in Kontakt kommendem heißem Wasser vorgeheizt wird. Die bevorzugte Wassertemperatur liegt im Bereich von 60° +/- 15° C und besser Bereich von 90° +/- 10° C. Typischerweise wird eine Conchierbehälter-Zeit von etwa 60 bis 120 Minuten gewählt. Die Conchierwerkzeuge laufen dabei vorzugsweise mit Geschwindigkeiten von unter 20 Umdrehungen/min um.

Nach Ablauf dieser Zeit ist eine bei richtiger Einstellung der Vorzerkleinerungs- und Conchierparameter eine Restfeuchtigkeit des conchierten Gutes im Bereich von 0, 5 % festzustellen, +/-20%.

Durch ein solches Conchieren bzw. Vermischen unter dem Einfluss von Vakuum werden die bis dahin noch intakten Zellwände der aus dem Vorzerkleinerer bezogenen Masse aufgebrochen. Auf diese Art und Weise werden in den Zellen bislang noch gespeicherten und daher immobil gehaltenen Fette und Öle freigesetzt. Gleichzeitig verringert sich die Restfeuchte. Letzteres, weil auch das Zellwasser mobilisiert wird. Dieses geht dann aber alsbald in die Gasphase über, anders als die mobilisierten Öle und Fette.

Dadurch nimmt die Viskosität der Masse stark ab. Die bisher teigige-zähe Masse wird deutlich dünnflüssiger. Ihre Pumpfähigkeit entsprechend zu. Nach den Feststellungen der Erfinder ist für diesen Effekt nicht das Vakuum allein verantwortlich. Vielmehr scheint den entscheidenden Effekt die Kombination aus Vakuum und der permanenten, länger (meist mehr als 45 min) andauernden Agitation der Masse durch den Mischer oder die Conchiervorrichtung auszumachen.

Nachdem das Conchieren beendet ist liegt eine Masse vor die schon so fließfähig ist, dass sie mithilfe einer Dickstoffpumpe in einen Vorratsbehälter gepumpt werden kann. Kolbenpumpen sind hier nun schon möglich, aber noch nicht das ideale Mittel der Wahl. Als ideal hat sich der Einsatz Exzenterschneckenpumpe erwiesen, etwa der Nemo-Baureihe der Fa. Netzsch. Wie dem auch sei, auf Grund ihrer nun verbesserten Pumpfähigkeit kann die durch das Mischen bzw. Conchieren unter Vakuum und eventueller, moderater Heizwärmeapplikation entstandene Masse durch eine Rührwerkskugelmühle 11 gepumpt werden.

Idealerweise kommt hierzu eine Rührwerkskugelmühle des Typs Masterrefiner^{®} zum Einsatz, wie sie die Firma Netzsch am Markt vertreibt. Am geeignetsten haben sich - bei dieser oder anderen Rührwerkskugelmühlen - Mahlkugeln mit einem Durchmesser im Bereich zwischen 2 mm und 8 mm erwiesen. Die Verweildauer der Masse in der Rührwerkskugelmühle liegt sinnvollerweise im Bereich von 2 bis 4 Stunden, dabei wird die Rührwerkskugelmühle meist mehrfach durchlaufen.

Auf diese Art und Weise kann in der Rührwerkskugelmühle ein Produkt, d.h. eine Ansetzmasse erreicht werden, dessen bzw. deren durchschnittliche Partikelgröße überwiegend im Bereich zwischen 1,5/100 mm und 10/100 mm liegt. Dadurch werden nun noch die letzten Reste an bisher noch gespeicherten Fetten und Ölen mobilisiert. So entsteht eine gut pumpfähige Ansatzmasse. Deren mit dem Rotationsviskosimeter zu messende Viskosität liegt vorzugsweise (und ohne Zugabe von Fremdstoffen) im Bereich von 1,00E+2 cP bis 1,00E+4 cP. Eine solche Ansatzmasse kann ohne weiteres mit den in der Lebensmittelindustrie üblichen Dosierpumpen weiterverarbeitet und dem Endprodukt präzise zudosiert werden.

Gesagt sei an dieser Stelle, dass der vorgenannte Vorratsbehälter 12 nicht zwingend erforderlich ist. Er ist aber vorteilhaft. Denn er ermöglicht es die Rührwerkskugelmühle 11 einen längeren Zeitraum zu betreiben, als nur für den kurzen Zeitraum in dem die nächste Charge aus der Conchiervorrichtung 8 entladen wird.

Für größere Durchsatzmengen wird das bisher geschilderte Verfahren dadurch modifiziert, dass zwei oder mehrere Horizontalmischer bzw. Conchiervorrichtungen 8 parallel betrieben werden. Auf diese Art und Weise ist durch Verwendung eines entsprechend großen Vorratsbehälters 12 sicherstellbar, dass die Vorrichtung zur Feinvermahlung, also die Rührwerkskugelmühle 11 bzw. der der Masterrefiner durchgehend beschickt werden kann. Dies deshalb, da immer wechselweise einer der beiden Horizontalmischer bzw. eine der beiden Conchiervorrichtungen beladen werden kann, während der oder die andere parallel arbeitet um dann kurzfristig weiteres Produkt zur Verfügung zu stellen, das der Rührwerkskugelmühle zugeführt werden kann.

Ein weiteres Ausführungsbeispiel weitestgehend genauso aufgebaut wie die bisher geschilderten Ausführungsbeispiele

Es unterscheidet sich von diesen aber, dass in der Conchiervorrichtung kein Vakuum appliziert wird. Stattdessen wird ihr Conchierbehälter Heißluft beschickt. Die Heißluft hat eine Sub-Maillardtemperatur idealerweise um die 90° C. Sie kommt unmittelbar mit der Masse in Berührung. Sie dient also nicht nur der indirekten Beheizung des Conchierbehältes.

Die Verweilzeit im Conchierbehälter liegt in diesem Fall etwas höher als bei der Vakuumapplikation. Sie beträgt dann vielfach etwa 90 bis 180 Minuten.

### BEZUGSZEICHENLISTE

1 Förderer
2 Vorzerkleinerer
3 Kegelmahlwerk
4 Brechkegel
5 Brechspalt
6 Brechring
7 Mahlscheibe
8 Horizontalmischer bzw. Conchiervorrichtung
9 Conchierbehälter
10 Conchierwerkzeug
11 Rührwerkskugelmühle
12 Vorratsbehälter bzw. process tank
13 Vakuumpumpe bzw. vacuum pump
A Radialbeschleunigung
B Grobmahlung
C Kühlen
D Auslassrotor

## Patentansprüche

1. Verfahren zur Zubereitung einer pumpfähigen Ansatzmasse aus Steinfrucht- oder/und Nussfruchtsamen, insbesondere Nüssen, Mandeln oder Nuss- und Mandelgemischen, mit folgenden Schritten:
zunächst erfolgt ein Aufbrechen der bereits ausgesteinten oder ausgeschalten Steinfrucht- oder Nussfruchtsamen durch Vorzerkleinerung;
anschließend erfolgt eine Heißluft- und/oder Vakuumexposition der durch die Vorzerkleinerung entstandenen Masse zum Zwecke des Aufbrechens ihrer Zellen in einer Conchiervorrichtung (8) mit einem zumindest im Wesentlichen abgeschlossenen Conchierbehälter (9) und einem darin bevorzugt an einer horizontalen Welle umlaufenden, meist mehrarmigen Conchierwerkzeug;
hieran schließt sich eine einstufige oder mehrstufige Weitervermahlung der durch die Vorzerkleinerung und die Heißluft- und/oder Vakuumexposition entstandenen Masse zu einer pumpfähigen Ansatzmasse an.

2. Verfahren zur Zubereitung einer pumpfähigen Masse aus Steinfrucht- oder/und Nussfruchtsamen nach Anspruch 1, **dadurch gekennzeichnet, dass** die Vorzerkleinerung eine Vorvermahlung ist, die bevorzugt ein Vorvermahlgut hervorbringt, welches im Wesentlichen oder zumindest überwiegend eine mittlere Partikelgröße im Bereich von 4/100 mm bis 30/100 mm aufweist.

3. Verfahren zur Zubereitung einer pumpfähigen Masse aus Steinfrucht- oder/und Nussfruchtsamen nach Anspruch 1 oder 2, **dadurch gekennzeichnet, dass** die Heißluft- und/oder Vakuumexposition, insbesondere über die Expositionsdauer, so gesteuert wird, dass die Restfeuchte auf unter 2,1% und bevorzugt auf unter 1,85% reduziert wird, idealerweise auf 0,5% +/- 0,2%

4. Verfahren zur Zubereitung einer pumpfähigen Masse aus Steinfrucht- oder/und Nussfruchtsamen nach Anspruch 1 oder 2, **dadurch gekennzeichnet, dass** während der Heißluftexposition die Lufttemperatur der zum Trocknen eingesetzten Heißluft im Bereich von oberhalb von 60° und idealerweise im Bereich von oberhalb 75° liegt.

5. Verfahren zur Zubereitung einer pumpfähigen Masse aus Steinfrucht- oder/und Nussfruchtsamen nach Anspruch 1, 2 oder 3, **dadurch gekennzeichnet, dass** während der Heißluftexposition die Lufttemperatur der zum Trocknen eingesetzten Heißluft im Bereich von unterhalb von 100° und idealerweise im Bereich von unterhalb 90° liegt.

6. Verfahren zur Zubereitung einer pumpfähigen Masse aus Steinfrucht- oder/und Nussfruchtsamen nach einem der vorhergehenden Ansprüche, **dadurch gekennzeichnet, dass** während der Vakuumexposition zum Trocknen ein Vakuum mit einen Absolutdruck von weniger als 0,25 bar und bevorzugt im Bereich von 0,1 bar angelegt wird.

7. Verfahren zur Zubereitung einer pumpfähigen Masse aus Steinfrucht- oder/und Nussfruchtsamen nach einem der vorhergehenden Ansprüche, **dadurch gekennzeichnet, dass** bei der Vorzerkleinerung und im Regelfall auch bei der Weitervermahlung kein Wasser, Öl oder sonstiger Steinfrucht- oder Nussfruchtsamenfremder Stoff zugesetzt wird.

8. Verfahren zur Zubereitung einer pumpfähigen Masse aus Steinfrucht- oder/und Nussfruchtsamen nach einem der vorhergehenden Ansprüche, **dadurch gekennzeichnet, dass** die Vorzerkleinerung durch eine Vorzerkleinerungsvorrichtung (2) erfolgt, die einen mit einer - bevorzugt vertikal angeordneten - Welle umlaufenden Brechkegel (4) aufweist, der in unter Ausbildung eines Brechspalts (5) einem stationären Brechring (6) umläuft, wobei die den Brechspalt (5) zwischen sich ausbildenden Flächen typischerweise mit mitnehmerbildenden Profilierung zum Einzug der vorzuzerkleinernden Steinfrucht- oder Nussfruchtsamen in den Brechspalt (5) versehen sind.

9. Vorrichtung zur Durchführung des Verfahrens nach einem der vorhergehenden Ansprüche, **dadurch gekennzeichnet, dass** die Vorrichtung einen Vorzerkleinerer (2), eine Conchiervorrichtung (8), einen Heißluft- oder Vakuumapplikator (13) und eine Feinvermahlungseinrichtung (11) umfasst.

## Claims

1. A method for preparing a pumpable slurry from stone fruit and/or nut seeds, in particular nuts, almonds, or mixtures of nuts and almonds, with the following steps:
first, the stone fruit or nut seeds, which have already been pitted or shelled, are broken down by pre-crushing;
subsequently, the mass produced by the pre-crushing is exposed to hot air and/or vacuum for the purpose of breaking open its cells in a conching device (8) comprising an at least substantially enclosed conching vessel (9) and a conching tool, preferably rotating on a horizontal shaft within it, typically having multiple arms;
this is followed by a single-stage or multi-stage further grinding of the mass produced by the pre-crushing and the hot air and/or vacuum exposure to form a pumpable mixture.

2. Method for preparing a pumpable mixture from stone fruit and/or nut seeds according to claim 1, **characterized in that** the pre-crushing is a pre-grinding process that preferably produces a pre-ground material having essentially or at least predominantly a mean particle size in the range of 4/100 mm to 30/100 mm.

3. A method for preparing a pumpable mass from stone fruit and/or nut seeds according to claim 1 or 2, **characterized in that** the hot air and/or vacuum exposure, in particular via the exposure duration, is controlled such that the residual moisture is reduced to below 2.1% and preferably to below 1.85%, ideally to 0.5% ± 0.2%

4. A method for preparing a pumpable mass from stone fruit and/or nut seeds according to claim 1 or 2, **characterized in that** during the hot air exposure, the air temperature of the hot air used for drying is in the range of above 60°C and ideally in the range of above 75°C.

5. A method for preparing a pumpable mass from stone fruit and/or nut seeds according to claim 1, 2, or 3, **characterized in that** during hot air exposure, the temperature of the hot air used for drying is in the range of below 100°C and ideally in the range of below 90°C.

6. A method for preparing a pumpable mass from stone fruit and/or nut seeds according to one of the preceding claims, **characterized in that** during vacuum exposure for drying, a vacuum with an absolute pressure of less than 0.25 bar and preferably in the range of 0.1 bar is applied.

7. A method for preparing a pumpable mass from stone fruit and/or nut seeds according to any of the preceding claims, **characterized in that** no water, oil, or other substances foreign to the stone fruit or nut seeds are added during pre-crushing and, as a rule, also during further grinding.

8. A method for preparing a pumpable mass from stone fruit and/or nut seeds according to one of the preceding claims, **characterized in that** the pre-crushing is performed by a pre-crushing device (2) comprising a crushing cone (4) rotating about a shaft-preferably arranged vertically- which rotates around a stationary crushing ring (6) to form a crushing gap (5), wherein the surfaces forming the crushing gap (5) between them are typically provided with a profiled surface acting as a driver to draw the stone fruit or nut seeds to be pre-crushed into the crushing gap (5).

9. Apparatus for carrying out the method according to one of the preceding claims, **characterized in that** the apparatus comprises a pre-crusher (2), a conching device (8), a hot air or vacuum applicator (13), and a fine grinding device (11).

## Revendications

1. Procédé de fabrication d'une bouillie pompable à partir de graines de fruits à noyau et/ou de noix, en particulier de noix, d'amandes ou de mélanges de noix et d'amandes, comprenant les étapes suivantes :
tout d'abord, les graines de fruits à noyau ou de noix, déjà dénoyautés ou décortiqués, sont broyés par pré-broyage;
ensuite, la masse obtenue par le pré-broyage est soumise à de l'air chaud et/ou au vide dans un dispositif de conchage (8), qui comprend une cuve de conchage (9) au moins essentiellement fermée et un outil de conchage qui tourne de préférence sur un arbre horizontal à l'intérieur de celle-ci et qui comporte typiquement plusieurs bras, afin de briser ses cellules ;
ceci est suivi d'un broyage supplémentaire en une ou plusieurs étapes de la masse obtenue par le pré-broyage et l'action de l'air chaud et/ou du vide, afin de former un mélange pompable.

2. Procédé de fabrication d'un mélange pompable à partir de de graines de fruits à noyau et/ou de noix selon la revendication 1, **caractérisé en ce que** le pré-broyage est un processus de pré-broyage qui produit de préférence un matériau pré-broyé présentant essentiellement ou au moins majoritairement une taille moyenne de particules comprise entre 4/100 mm et 30/100 mm.

3. Procédé de fabrication d'une pâte pompable à partir de de graines de fruits à noyau et/ou de noix selon la revendication 1 ou 2, **caractérisé en ce que** le traitement à l'air chaud et/ou sous vide, notamment en ce qui concerne la durée du traitement, est contrôlé de telle sorte que l'humidité résiduelle soit inférieure à 2,1 % et de préférence inférieure à 1,85 %, idéalement à 0,5 % ± 0,2 %

4. Procédé de fabrication d'une pâte pompable à partir de de graines de fruits à noyau et/ou de noix selon la revendication 1 ou 2, **caractérisé en ce que**, pendant le traitement à l'air chaud, la température de l'air chaud utilisé pour le séchage se situe dans une plage supérieure à 60 °C et, idéalement, dans une plage supérieure à 75 °C.

5. Procédé de fabrication d'une pâte pompable à partir de de graines de fruits à noyau et/ou de noix selon la revendication 1, 2 ou 3, **caractérisé en ce que**, pendant le traitement à l'air chaud, la température de l'air chaud utilisé pour le séchage se situe dans une plage inférieure à 100 °C et, idéalement, dans une plage inférieure à 90 °C.

6. Procédé de fabrication d'une pâte pompable à partir de de graines de fruits à noyau et/ou de noix selon l'une des revendications précédentes, **caractérisé en ce que**, pendant le traitement sous vide destiné au séchage, un vide présentant une pression absolue inférieure à 0,25 bar et de préférence comprise entre 0,1 bar est appliqué.

7. Procédé de fabrication d'une pâte pompable à partir de graines de fruits à noyau et/ou de noix selon l'une des revendications précédentes, **caractérisé en ce que**, pendant le pré-broyage et généralement aussi pendant le broyage ultérieur, aucune eau, aucune huile ni aucune autre substance étrangère aux graines de fruits à noyau ou de noix n'est ajoutée.

8. Procédé de fabrication d'une pâte pompable à partir de graines de fruits à noyau et/ou de noix selon l'une des revendications précédentes, **caractérisé en ce que** le pré-broyage est effectué par un dispositif de pré-broyage (2) qui comprend un cône de broyage (4) tournant autour d'un arbre - de préférence disposé verticalement -, lequel tourne autour d'une bague de broyage fixe (6) pour former une fente de broyage (5), les surfaces formant entre elles l'interstice de concassage (5) étant généralement pourvues d'une surface profilée agissant comme un entraîneur pour attirer les graines de fruits à noyau ou de noix dans l'interstice de concassage (5).

9. Dispositif destiné à la mise en œuvre du procédé selon l'une des revendications précédentes, **caractérisé en ce que** le dispositif comprend un pré-concasseur (2), un dispositif de conchage (8), une installation à air chaud ou sous vide (13) et un dispositif de broyage fin (11).
